# EUROPÄISCHE PATENTANMELDUNG

(11) **EP 3 893 394 A1**
(43) Veröffentlichungstag der Anmeldung: **13.10.2021**
(21) Anmeldenummer: 21167310.8
(22) Anmeldetag: 08.04.2021
(51) Int. Cl.: H03K 17/96, G06F 3/00

(54) **BEDIENVORRICHTUNG UND MEDIZINISCHES GERÄT**

(30) Priorität: 09.04.2020 DE 102020110056
(71) Anmelder: Karl Storz SE & Co. KG, 78532 Tuttlingen (DE)
(72) Erfinder: Storz, Klaus, 78532 Tuttlingen (DE); Geißer, Romana, 78532 Tuttlingen (DE); Sauter, Achim, 78532 Tuttlingen (DE)
(74) Vertreter: Weidner Stern Jeschke

(57) **Zusammenfassung**

Die Erfindung betrifft eine Bedienvorrichtung zum Interagieren mit einem Benutzer mit einer transparenten Deckeinrichtung, einer Trägereinrichtung mit einer Sensoraussparung und einem Druckschalter, wobei die transparente Deckeinrichtung eine Bedienfläche und eine der Bedienfläche gegenüberliegende Unterseite ausbildet und die Trägereinrichtung auf oder an der Unterseite angeordnet ist und in der Sensoraussparung der Druckschalter derart verortet und ausgerichtet ist, dass eine Benutzereingabe mittels eines Fingers des Benutzers auf der Bedienfläche durch den Druckschalter detektierbar ist, wobei auf der Bedienfläche eine Fingermulde vorgesehen ist, welche oberhalb des Druckschalters angeordnet ist.

## Beschreibung

Die Erfindung betrifft eine Bedienvorrichtung zum Interagieren mit einem Benutzer, mit einer transparenten Deckeinrichtung, einer Trägereinrichtung mit einer Sensoraussparung und einem Druckschalter, wobei die transparente Deckeinrichtung eine Bedienfläche und eine der Bedienfläche gegenüberliegende Unterseite ausbildet und die Trägereinrichtung auf oder an der Unterseite angeordnet ist und in der Sensoraussparung der Druckschalter derart verortet und ausgerichtet ist, dass eine Benutzereingabe mittels eines Fingers des Benutzers auf der Bedienfläche durch den Druckschalter detektierbar ist, sowie ein medizinisches Gerät.

Um die Bedienoberfläche von medizinischen Geräten, wie Lichtquellen, Insufflatoren oder Pumpen, besser reinigen zu können, ist es gewünscht, dass Bedienelemente hinter einer Glasscheibe liegen. Das bedeutet, dass beispielsweise ein berührungssensitiver Bildschirm für Anzeigen und Eingaben und gegebenenfalls ein weiterer Bildschirm und ein Ein-/Ausschalter hinter einer Glasscheibe angeordnet sein sollten. Der Ein-/Ausschalter des Geräts kann dabei beispielsweise als Piezoschalter ausgebildet sein.

Gerade bei kompakten Geräten kann der Platz an der Bedienoberfläche begrenzt sein. In diesem Fall kann der Schalter ziemlich dicht neben dem berührungssensitiven Bildschirm liegen und es kann vorkommen, dass bei Betätigen des Schalters der benachbarte berührungssensitive Bildschirm (mit)bedient wird.

Aus der DE 10 2004 048 463 A1 ist beispielsweise ein Bedienfeld für ein Glas-Kochfeld bekannt, bei welchem eine Mehrzahl von Näherungssensoren unter einer Glasscheibe angeordnet sind. Weiterhin weist das Bedienfeld Schaltungsmechanismen gegen eine Fehlbedienung auf.

Die US 2020/0057467 A1 beschreibt ein Mobiltelefon, bei welchem ein biometrischer Sensor hinter dem Deckglas auf der Vorderseite des Mobiltelefons angeordnet ist.

Aufgabe der Erfindung ist es, den Stand der Technik zu verbessern.

Gelöst wird die Aufgabe durch eine Bedienvorrichtung zum Interagieren mit einem Benutzer, mit einer transparenten Deckeinrichtung, einer Trägereinrichtung mit einer Sensoraussparung und einem Druckschalter, wobei die transparente Deckeinrichtung eine Bedienfläche und eine der Bedienfläche gegenüberliegende Unterseite ausbildet und die Trägereinrichtung auf oder an der Unterseite angeordnet ist und an der Sensoraussparung der Druckschalter derart verortet und ausgerichtet ist, dass eine Benutzereingabe mittels eines Fingers des Benutzers auf der Bedienfläche durch den Druckschalter detektierbar ist, wobei auf der Bedienfläche eine Fingermulde vorgesehen ist, welche derart oberhalb des Druckschalters angeordnet ist, dass ein Drücken der Fingermulde mittels eines Fingers des Benutzers einen Schaltvorgang realisiert.

Dadurch kann gerade bei knappen Platzverhältnissen ein unterhalb einer Glasfläche angeordneter Drucksensor definiert geschaltet werden. Das hat weiterhin zur Folge, dass ein berührungssensitiver Bildschirm, wie beispielsweise ein Touchscreen, welcher in direkter Nachbarschaft zu dem Druckschalter angeordnet ist, nicht versehentlich bedient wird, da die Fingermulde den Finger eines Bedieners entsprechend führt und definiert zum Druckschalter ausrichtet.

Somit kann vorteilhafterweise ein Druckschalter näher an einen Touchscreen angeordnet oder entsprechend ein Touchscreen etwas größer ausgestaltet werden.

Folgendes Begriffliche sei erläutert:
Eine "Bedienvorrichtung" ist beispielsweise ein Bedienterminal oder ein sonstiges Eingabemittel, über welches ein Bediener einzelne Steuerelemente, wie beispielsweise einen Druckschalter oder einen Touchscreen, ansteuern kann. Mithin dient die Bedienvorrichtung zur Eingabe und/oder Ausgabe von Daten.

Unter "Interagieren" ist insbesondere jeglicher berührungsbehaftete Austausch von Daten und/oder Informationen zu verstehen. Dies kann auch ein Schalten eines Schalters umfassen. Auch ist darunter das Anzeigen von Informationen an einen Bediener oder Benutzer mit umfasst.

Die "transparente Deckeinrichtung" schützt insbesondere unterhalb der transparenten Deckeinrichtung befindliche Anzeige- und Schaltelemente und kann im Allgemeinen gut, beispielsweise mit Desinfektionsmitteln, gereinigt werden. Die transparente Deckeinrichtung kann dabei beispielsweise aus einem transparenten festen Kunststoff oder auch aus einem Glas gefertigt sein. Bei einer solchen transparenten Abdeckung, beispielsweise einem Glas, reicht eine Verformung im Bereich einiger Mikrometer, um beispielsweise das Schaltelement zu betätigen.

Mittels der "Trägereinrichtung" kann der transparenten Deckeinrichtung durch eine abstützende Funktion ein Halt gegeben werden. Zusätzlich können an oder in der transparenten Deckeinrichtung Bauteile, wie beispielsweise Schalter, Stecker oder auch Bildschirme, eingepasst oder angeordnet und durch die Trägereinrichtung gefasst sein. Insbesondere weist die Trägereinrichtung eine "Sensoraussparung" auf. Zum einen kann die durch die Sensoraussparung gebildete Kante ein Kräfteübertragen an andere Stellen der transparenten Deckeinrichtung verhindern oder vermindern und weiterhin den Druckschalter aufnehmen.

Dabei ist der "Druckschalter" (auch als "Drucksensor" bezeichnet) insbesondere ein Piezoschalter, welcher räumliche Veränderungen von einigen Mikrometern aufgrund eines Betätigens mit einem Finger definiert detektieren kann. Eine solches Betätigen im Bereich einiger Mikrometer ist auch durch das vergleichsweise geringe elastische Verhalten der transparenten Abdeckung möglich. Um den Druckschalter besser erkennbar zu machen, kann der Druckschalter ein Leuchtelement aufweisen, welches derart angeordnet ist, dass das Leuchtelement durch einen Benutzer durch die transparente Deckeinrichtung hindurch erkennbar ist. Insbesondere ist das Leuchtelement kreis- oder entsprechend ringförmig. Das Leuchtelement kann als LED ausgestaltet sein. Zudem kann das Leuchtelement als ein um einen Schaltpunkt liegender beleuchteter Ring ausgestaltet sein. Auch können Piezoschalter mit bereits integriertem Leuchtring eingesetzt werden.

Mithin weist die transparente Deckeinrichtung eine "Bedienfläche" auf, auf welcher der Benutzer, beispielsweise mittels seines Fingers, den Druckschalter betätigen oder gegebenenfalls eine Auswahl auf einem Touchscreen treffen kann.

Gegenüber der Bedienfläche weist die transparente Deckeinrichtung eine Unterseite auf, an welcher insbesondere die Trägereinrichtung, beispielsweise mittels Kleber, aufgebracht und angeordnet ist. An der Unterseite können der Druckschalter oder auch Bildschirme, sensitive Bildschirme, Stecker oder dergleichen angeordnet sein.

Eine "Fingermulde" ist insbesondere eine Vertiefung auf der Bedienfläche der transparenten Deckeinrichtung. Dabei kann die Fingermulde so ausgestaltet sein, dass eine Fingerspitze eines Benutzers aufgenommen werden kann. Mithin vermittelt die Fingermulde einem Benutzer ein haptisches Signal, sodass auch einem Benutzer vermittelt wird, dass er einen darunterliegenden Druckschalter definiert betätigen kann.

Vorteilhafterweise wird durch die Fingermulde die Kraft eines Fingers, welcher den Druckschalter betätigt, definierter übertragen. Somit wird das Auslösen des Druckschalters sichergestellt. Zudem kann aufgrund dessen, dass an der Stelle der Fingermulde weniger stabilisierendes Material der transparenten Deckeinrichtung vorhanden ist, mittels eines geringeren Drucks der Drucksensor oder entsprechend der Druckschalter angesprochen werden.

Aufgrund dessen, dass die Fingermulde auf der Bedienfläche angeordnet ist, kann insbesondere ein eineindeutiges Schalten dadurch sichergestellt werden, dass der Druckschalter auf der Unterseite gegenüberliegend der Fingermulde angeordnet ist, sodass sich die Fingermulde oberhalb des Druckschalters befindet. Mithin befindet sich die Fingermulde auch dann oberhalb des Druckschalters, sofern mittels eines Fingers in der Fingermulde der Druckschalter noch bei normaler Handhabung definiert betätigbar ist.

Ein besonders sicheres Betätigen des Druckschalters ist insbesondere dann gewährleistet, wenn eine Dicke der Fingermulde zwischen 30 % und 70 %, insbesondere zwischen 40 % und 60 % oder insbesondere zwischen 45 % und 55 %, einer Dicke der transparenten Deckeinrichtung beträgt. Gute Ergebnisse werden beispielsweise dann erreicht, wenn die transparente Deckeinrichtung eine Dicke von 3 - 5 mm und die Fingermulde einen Tiefenwert von 1,5 - 2,5 mm aufweist.

Die "Dicke der transparenten Deckeinrichtung" ist insbesondere der minimale Abstand gegenüberliegender Punkte der transparenten Deckeinrichtung außerhalb der Fingermulde also mithin der Abstand von der Bedienfläche zur Unterseite.

Der "Tiefenwert der Fingermulde" wird insbesondere an dem tiefsten Punkt von der Bedienfläche aus gesehen bestimmt. Somit ist der Tiefenwert zugleich an dem Punkt zu bestimmen, welcher für die Fingermulde ein Minimum in der Dicke der Deckeinrichtung darstellt.

In einer weiteren Ausführungsform ist die Fingermulde kugelsegmentförmig oder ellipsoidsegmentförmig ausgestaltet. Diese Formen stellen insbesondere eine sehr gute Aufnahme für eine Fingerkuppe eines Benutzers dar.

Unter "kugelsegmentförmig" ist eine Form zu verstehen, wenn sie in ihrer Form einen geschnittenen Teil einer Kugel darstellt. Dies kann jeden beliebigen Schnitt umfassen und somit ist unter kugelsegmentförmig auch die Form einer Halbkugelschale zu verstehen. Analoges gilt für ellipsoidförmige Fingermulden, wobei die Schnitte parallel oder orthogonal zur Längsachse eines ellipsoidförmigen Körpers erfolgen.

Um für den Finger eines Benutzers optimal ausgerichtet zu sein, kann die Fingermulde einen Maximaldurchmesser mit einem Wert zwischen 1,0 cm und 3,0 cm aufweisen.

Ein Maximaldurchmesser ist der größte Abstand, der zwischen den Rändern der Fingermulde bestimmbar ist.

Um besonders sensitiv Drücke auf der Fingermulde detektieren zu können, ist der Druckschalter als Piezoschalter ausgestaltet. Dabei ist ein "Piezo" ein Kristall - und wird somit auch häufig Piezokristall genannt -, welcher beim Beaufschlagen von Druck eine Spannung erzeugt. Somit kann der Piezoschalter den Druck in eine Spannung umwandeln, welche entsprechend elektrisch und elektronisch aufbereitet wird und zum Schalten, beispielsweise zum Ein- und/oder Ausschalten eines Gerätes, verwendet wird.

In einer weiteren Ausführungsform weist der Druckschalter ein festes Gehäuse auf.

Dieses Gehäuse ist insbesondere ein Zusatzgehäuse, welches beispielsweise den Piezoschalter oder entsprechend den Druckschalter aufnimmt und außen stabilisiert.

Vorliegend hat sich überraschender Weise gezeigt, dass sich durch das feste (Zusatz-)Gehäuse der Druck der Finger auf der Fingermulde noch besser bestimmen lässt. Mithin wurden bei Tests weniger Fehlbedienungen des Druckschalters festgestellt. Besonders vorteilhaft ist dies insbesondere dann, wenn das feste Gehäuse mit einem Rand des Gehäuses mit der Unterseite der Deckeinrichtung, insbesondere mittels einer Klebeschicht, verbunden ist. Insbesondere umfasst das Zusatzgehäuse den Druckschalter mantelförmig, wobei auch eine zur transparenten Deckeinrichtung geöffnete Kapselung des Druckschalters realisierbar ist. Besonders vorteilhaft stützt das Zusatzgehäuse mit seinem Rand die transparente Deckeinrichtung dadurch ab, dass der Rand mit der transparenten Deckeinrichtung verbunden ist. Das Zusatzgehäuse kann beispielsweise aus einem Metall oder einer Metalllegierung oder auch aus einem Kunststoff gefertigt sein. Insbesondere ist das Zusatzgehäuse mit dem Druckschalter verbunden und bildet mit dem Druckschalter eine Einheit. Eine Randstärke weist insbesondere einen Wert zwischen 0,5mm und 5,0mm auf, jedoch können auch größere Randstärken bis zu 2cm realisiert werden. Insbesondere kann das Zusatzgehäuse auch als Gehäuse in der Trägereinrichtung integriert sein, also beispielsweise aus einem Grundmaterial der Trägereinrichtung gefräst sein.

Das feste Gehäuse kann mit der Unterseite der Deckeinrichtung, insbesondere mittels einer Klebeschicht , verbunden sein, wobei insbesondere das Gehäuse unterhalb der Fingermulde angeordnet ist.

Um den Hygienestandards im medizinischen Bereich gerecht zu werden und um die Oberfläche der Bedienvorrichtung mit Desinfektionsmitteln behandeln zu können, ist die transparente Deckeinrichtung als eine Glasschicht oder entsprechend als Glasscheibe ausgestaltet. Dünnes, industriell verfügbares Glas weist dabei insbesondere trotz eines relativ steifen Verhaltens genügend Elastizität auf, um das Betätigen beispielsweise eines Piezo-Schalters zu ermöglichen. Gleiches gilt auch für entsprechende transparente Abdeckungen aus einem Kunststoff.

"Glas" ist dabei insbesondere ein amorpher Feststoff, welcher hauptsächlich Siliziumdioxid umfasst.

In einer weiteren Ausführungsform weist die Trägereinrichtung eine Bildschirmaussparung und/oder weitere Bildschirmaussparungen und/oder eine Geräteanschlussaussparung und/oder weitere Geräteanschlussaussparungen auf. Innerhalb der Bildschirmaussparung oder der weiteren Bildschirmaussparung kann somit ein Bildschirm oder können somit weitere Bildschirme, wie beispielsweise Touch-Bildschirme, eingebracht werden. Aber auch einzelne Anzeigeelemente und dergleichen werden vorliegend als Bildschirm verstanden.

"Geräteanschlussaussparung" ist insbesondere eine Aussparung für einen Stecker, über welchen beispielsweise Energie, Daten, Fluide, Gase und dergleichen ausgetauscht werden können. Als Stecker kann auch eine Dose zu verstehen sein, wobei im Allgemeinen Dose und Stecker ein zueinander korrespondierendes Stecksystem ausbilden. Für den Fall, dass es sich um eine Geräteanschlussaussparung oder mehrere Geräteanschlussaussparungen handelt, kann die transparente Deckeinrichtung ebenfalls eine Aussparung aufweisen, sodass an der Bedienfläche eine entsprechende korrespondierende Geräteanschlusseinheit eingebracht werden kann.

Somit kann die Bedienungseinrichtung einen weiteren Bildschirm, insbesondere berührungssensitiven Bildschirm, wie beispielsweise einen Touchscreen, oder weitere Bildschirme aufweisen.

In einem weiteren Aspekt wird die Aufgabe durch ein medizinisches Gerät gelöst, welches eine zuvor beschriebene Bedienvorrichtung aufweist.

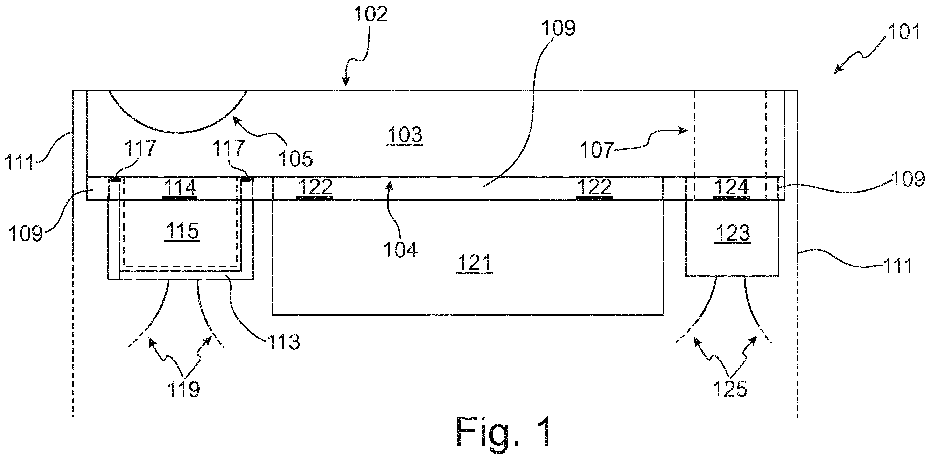

Im Weiteren wird die Erfindung anhand eines Ausführungsbeispiels näher erläutert. Es zeigt
- Figur 1: eine stark schematische Seitenschnittdarstellung eines Bedienterminals eines Medizingeräts.

Ein Bedienterminal 101 eines Medizingeräts weist eine 4 mm dicke Glasscheibe 103 mit einer Oberseite 102 und eine Unterseite 104 und ein Terminalgehäuse 111 auf. An der Unterseite 104 ist eine aus Aluminium gefertigte Trägerplatte 109 mit einer Sensoraussparung 114, einer Bildschirmaussparung 122 und einer Steckeraussparung 124 mittels Kleben direkt mit der Glasscheibe 103 verbunden.

In der Sensoraussparung 114 ist ein Piezoschalter 115, welcher in einem stabilisierenden Zusatzgehäuse 113 angeordnet ist, auf der Unterseite 104 mittels einer Klebeschicht 117 angebracht, sodass die Klebeschicht 117 die Unterseite 104 mit einem ringförmigen Rand des Zusatzgehäuses 113 verbindet.

Das stabilisierende Zusatzgehäuse 113 ist topfförmig ausgestaltet und zur Unterseite 104 geöffnet und kapselt somit den Piezoschalter 115. In einer Alternative kann das Zusatzgehäuse dabei auch an anderen Stellen geöffnet sein, beispielsweise mittels zusätzlicher Durchbrüche oder Öffnungen. Eine Mantelwandstärke des Zusatzgehäuses 113 ist 1 mm dick, wobei hier in Alternativen auch größere Mantelwandstärken umgesetzt werden können.

Direkt oberhalb des Piezoschalters 115 weist die Glasscheibe 103 eine muldenartige, kugelsegmentförmige Vertiefung 105 auf. Die Mulde 105 dient vorliegend als Fingermulde und weist einen Maximaldurchmesser von 1,5 cm bis 3,0 cm und eine Tiefe von 2 mm auf.

In einem seitlichen Abstand zum Zusatzgehäuse von 1 mm ist in der Bildschirmaussparung 122 ein berührungssensitiver Bildschirm 121 eingebracht. Dieser berührungssensitive Bildschirm 121 ist mit der Trägerplatte 109 verbunden und kontaktiert mit seiner Oberfläche die Unterseite 104 der Glasscheibe 103.

Seitlich neben dem berührungssensitiven Bildschirm 121 ist auf der Unterseite 104 der Glasscheibe 103 in der Steckeraussparung 124 und darunterliegend ein Steckergehäuse 123 angeordnet. Zusätzlich weist die Glasscheibe 103 eine kreisrunde Steckeraussparung 107 auf.

Im Folgenden wird die Bedienung des Bedienterminals besprochen.

Ein Benutzer schaltet mittels des Bedienterminals 101 ein medizinisches Gerät dadurch an, dass er mit seinem Finger in die Fingermulde 105 eingreift und in Richtung des Piezoschalters 115 einen Druck ausübt. Dieser Druck wird von dem Piezoschalter 115 detektiert und über zugehörige Piezoanschlüsse 119 an eine Elektronik (nicht dargestellt) weitergeleitet, welche daraufhin den berührungssensitiven Bildschirm 121 aktiviert.

Zusätzlich verknüpft der Bediener das Bedienterminal mit einem medizinischen Instrument (nicht dargestellt) mittels eines Steckers, welcher über die Oberseite 102 der Glasscheibe 103 durch die Steckeraussparung 107 in das korrespondierende Steckergehäuse 123 eingebracht wird. Die entsprechenden Signale werden über die Steckeranschlüsse 125 an die Elektronik weitergeleitet.

Anschließend erfolgt eine Bedienerführung mittels des berührungssensitiven Bildschirms, wobei oberhalb des berührungssensitiven Bildschirms 121 durch Berühren der Oberseite 102 der Glasscheibe 103 Menüpunkte ausgewählt und angesteuert werden.

Daraufhin erfolgt die Behandlung eines Patienten.

Nach Abschluss der Behandlung wird das Bedienterminal 101 dadurch ausgeschaltet, dass der Bediener seine Fingerkuppe in die Fingermulde 105 einführt und einen Druck in Richtung Unterseite 104 der Glasscheibe 103 ausübt. Dieser Druck wird durch den Piezoschalter 115 detektiert und über die Piezoanschlüsse 119 an die Elektronik weitergeleitet, woraufhin der berührungssensitive Bildschirm 121 ausgeschaltet wird.

Abschließend wird die Oberseite 102 der Glasscheibe 103 mit einem Desinfektionsmittel behandelt, sodass die Oberseite 102 der Glasscheibe steril ist.

### Bezugszeichenliste

101 Bedienterminal eines Medizingeräts
102 Oberseite der Glasscheibe
103 Glasscheibe
104 Unterseite der Glasscheibe
105 Fingermulde
107 Steckeraussparung
109 Trägerplatte
111 Terminalgehäuse
113 Zusatzgehäuse
114 Sensoraussparung
115 Piezoschalter
117 Klebeschicht
119 Piezoanschlüsse
121 berührungssensitiver Bildschirm
122 Bildschirmaussparung
123 Steckergehäuse
124 Steckeraussparung
125 Steckeranschlüsse

## Patentansprüche

1. Bedienvorrichtung (101) zum Interagieren mit einem Benutzer mit einer transparenten Deckeinrichtung (103), einer Trägereinrichtung (109) mit einer Sensoraussparung (114) und einem Druckschalter (115), wobei die transparente Deckeinrichtung eine Bedienfläche (102) und eine der Bedienfläche gegenüberliegende Unterseite (104) ausbildet und die Trägereinrichtung auf oder an der Unterseite angeordnet ist und in der Sensoraussparung der Druckschalter derart verortet und ausgerichtet ist, dass eine Benutzereingabe mittels eines Fingers des Benutzers auf der Bedienfläche durch den Druckschalter detektierbar ist, **dadurch gekennzeichnet, dass** auf der Bedienfläche eine Fingermulde (105) vorgesehen ist, welche derart oberhalb des Druckschalters angeordnet ist, dass ein Drücken der Fingermulde mittels eines Fingers des Benutzers einen Schaltvorgang realisiert.

2. Bedienvorrichtung nach Anspruch 1, **dadurch gekennzeichnet, dass** ein Tiefenwert der Fingermulde zwischen 30% und 70%, insbesondere zwischen 40% und 60% oder insbesondere zwischen 45% und 55%, einer Dicke der transparenten Deckeinrichtung beträgt.

3. Bedienvorrichtung nach einem der vorherigen Ansprüche, **dadurch gekennzeichnet, dass** die Fingermulde kugelsegmentförmig oder ellipsoidsegmentförmig ist.

4. Bedienvorrichtung nach einem der vorherigen Ansprüche, **dadurch gekennzeichnet, dass** ein Maximaldurchmesser der Fingermulde einen Wert zwischen 1,0cm und 3,0cm, beispielsweise 2,0cm, aufweist.

5. Bedienvorrichtung nach einem der vorherigen Ansprüche, **dadurch gekennzeichnet, dass** der Druckschalter ein Piezoschalter ist.

6. Bedienvorrichtung nach einem der vorherigen Ansprüche, **dadurch gekennzeichnet, dass** der Druckschalter ein festes Gehäuse (113) aufweist.

7. Bedienvorrichtung nach Anspruch 6, **dadurch gekennzeichnet, dass** das feste Gehäuse mit der Unterseite der Deckeinrichtung, insbesondere mittels einer Klebeschicht (117) und/oder mittels einer mechanischen Verbindung, beispielsweise einer Rastverbindung, verbunden ist, wobei insbesondere das Gehäuse unterhalb der Fingermulde angeordnet ist.

8. Bedienvorrichtung nach einem der vorherigen Ansprüche, **dadurch gekennzeichnet, dass** die transparente Deckeinrichtung eine Glasschicht ist.

9. Bedienvorrichtung nach einem der vorherigen Ansprüche, **dadurch gekennzeichnet, dass** die Trägereinrichtung eine Bildschirmaussparung (122) und/oder weitere Bildschirmaussparungen und/oder eine Geräteanschlussaussparung (124) und/oder weitere Geräteanschlussaussparungen aufweist.

10. Bedienvorrichtung nach einem der vorherigen Ansprüche, **dadurch gekennzeichnet, dass** die Bedienvorrichtung einen weiteren Bildschirm, insbesondere berührungssensitiven Bildschirm, oder weitere Bildschirme aufweist.

11. Medizinisches Gerät, welches eine Bedienvorrichtung nach einem der vorherigen Ansprüche aufweist.
